# DEMANDE DE BREVET EUROPEEN

(11) **EP 4 632 642 A1**
(43) Date de publication de la demande: **15.10.2025**
(21) Numéro de dépôt: 25169856.9
(22) Date de dépôt: 10.04.2025
(51) Int. Cl.: G06Q 10/0631, G06Q 10/0639, G06Q 50/40

(54) **PROCÉDÉ DE CARACTÉRISATION DE ROUTES AÉRONAUTIQUES ET SYSTÈME DE CARACTÉRISATION ASSOCIÉ**

(30) Priorité: 12.04.2024 FR 2403808
(71) Demandeur: THALES, 92190 Meudon (FR)
(72) Inventeur: DURIEUX, Florence, 33700 MERIGNAC (FR); BERTHELOT, Bastien, 33700 MERIGNAC (FR); GRANIER, Lionel, 33700 MERIGNAC (FR)
(74) Mandataire: Lavoix

(57) **Abrégé**

La présente invention concerne un procédé de caractérisation de routes aéronautiques, chaque route aéronautique présentant un enchainement de vols effectués par un même opérateur, le procédé comprenant les étapes suivantes :
- acquisition (110) d'une pluralité de données d'évaluation relatives à une population d'opérateurs, les données d'évaluation étant déterminées à partir de données physiologiques des opérateurs ;
- prétraitement (140) des données d'évaluation ;
- acquisition (130) d'une pluralité de données de contexte particulier relatives au contexte particulier d'évaluation des opérateurs ;
- extraction (150) à partir des données de contexte particulier, de données sur des vols effectués par les opérateurs et identification d'une pluralité de routes aéronautiques correspondantes ;
- pour chaque route aéronautique identifiée, détermination (170) d'un indicateur de fatigue en fonction des données d'évaluation des opérateurs ayant effectué cette route.

## Description

La présente invention concerne un procédé de caractérisation de routes aéronautiques.

La présente invention concerne également un système de caractérisation mettant en œuvre un tel procédé.

L'invention se situe dans le domaine technique de l'évaluation de la fatigue d'opérateurs du domaine aéronautique pour améliorer la sécurité des vols.

Le problème général de l'invention à résoudre est d'optimiser la gestion du risque lié aux membres d'équipage en rapport à leur état de fatigue réel.

Dans l'état de la technique, la fatigue des équipages est généralement analysée lors de campagnes temporaires sur la base de questionnaires permettant de capturer la fatigue subjective, ou alors sur la base de déclarations de fatigue individuelles des équipages.

Il est également connu d'évaluer la fatigue des équipages sur la base de modèles biomathématiques qui sont destinés à lever des alertes ponctuelles et individuelles. Ces modèles ne permettent toutefois pas la contextualisation des données ni le croisement de différentes données à l'échelle d'une population.

Selon les méthodes connues de l'état de la technique, il est donc possible de déduire uniquement une fatigue subjective des équipages qui peut être biaisée par une pression culturelle ou d'entreprise. En effet, les équipages ont tendance généralement à sous-estimer leur fatigue.

Les informations fournies selon les méthodes de l'état de la technique ne permettent alors pas de déterminer la fatigue des équipages de manière fiable afin de planifier correctement les vols devant être effectués par ces équipages. Cela peut alors avoir des conséquences sur la sécurité de ces vols.

La présente invention a pour objectif de résoudre ce problème et de proposer des moyens permettant de planifier efficacement les vols tout en prenant en compte l'état de fatigue des opérateurs opérant ces vols.

Cela permet alors d'améliorer la sécurité de ces vols.

À cet effet, l'invention a pour objet un procédé de caractérisation de routes aéronautiques, chaque route aéronautique présentant un enchainement de vols effectués par un même opérateur, chaque vol étant défini par un aéroport de départ et un aéroport d'arrivée, le procédé comprenant les étapes suivantes :
- acquisition d'une pluralité de données d'évaluation relatives à une population d'opérateurs, les données d'évaluation étant déterminées à partir de données physiologiques des opérateurs ;
- prétraitement des données d'évaluation ;
- acquisition d'une pluralité de données de contexte particulier relatives au contexte particulier d'évaluation des opérateurs ;
- extraction à partir des données de contexte particulier, de données sur des vols effectués par les opérateurs et identification d'une pluralité de routes aéronautiques correspondantes ;
- pour chaque route aéronautique identifiée, détermination d'un indicateur de fatigue en fonction des données d'évaluation des opérateurs ayant effectué cette route.

Suivant d'autres aspects avantageux de l'invention, le procédé comprend l'une ou plusieurs des caractéristiques suivantes prises isolément ou suivant toutes les combinaisons techniquement possibles :
- les données d'évaluation comprennent au moins un type de données choisi dans le groupe comprenant:
   - niveau de fatigue objectif de l'opérateur ;
   - niveau de fatigue subjectif de l'opérateur ;
- le prétraitement des données d'évaluation comprend la mise en œuvre d'au moins l'un des éléments choisi dans le groupe comprenant :
   - normalisation du niveau de fatigue objectif ;
   - normalisation du niveau de fatigue subjectif ;
   - identification d'une classe de fatigue objective selon le niveau de fatigue objectif ;
   - identification d'une classe de fatigue subjective selon le niveau de fatigue subjectif ;
- la détermination de l'indicateur de fatigue pour chaque route aéronautique comprend la détermination d'une somme pondérée des niveaux de fatigue des opérateurs ayant effectué cette route ;
- les coefficients de pondération sont déterminés selon des règles prédéterminées ;
- une étape de corrélation de données acquises/déterminées lors de différentes phases de collecte réalisées en relation avec un même opérateur, chaque phase de collecte étant choisie parmi une phase initiale de collecte mise en œuvre avant la mission, une phase intermédiaire de collecte mise en œuvre pendant la mission et une phase finale de collecte mise en œuvre après la mission ;
- une étape acquisition d'une pluralité de données de contexte général relatives au contexte général d'évaluation des opérateurs, ledit étape de prétraitement comprenant en outre un prétraitement de ces données de contexte général ;
- les données de contexte général comprennent au moins un type de données choisi dans le groupe comprenant :
   - données relatives à l'environnement de l'opérateur ;
   - données physiologiques de l'opérateur ;
- le prétraitement des données de contexte général comprend la mise en œuvre d'au moins l'un des éléments choisi dans le groupe comprenant :
   - définition de la localisation de l'évaluation ;
   - définition de l'heure locale ;
   - identification d'une session matinale, normale ou tardive ;
   - identification du poste occupé par l'opérateur ;
   - extrapolation des informations relatives au sommeil de l'opérateur ;
- l'étape de détermination d'un indicateur de fatigue pour chaque route aéronautique comprend en outre la détermination de différents indicateurs de fatigue associées à cette route aéronautique en fonction de différentes données de contexte général formant un ou plusieurs critères de filtrage ;
- une étape de détermination d'un indicateur de fatigue pour chaque vol d'une même route aéronautique en fonction de niveaux de fatigue objectifs/subjectifs déterminés pour différents opérateurs pour ce vol.

L'invention a également pour objet un système de caractérisation de routes aéronautiques, comprenant des moyens techniques configurés pour mettre en œuvre le procédé tel que défini précédemment.

L'invention sera mieux comprise à la lecture de la description qui va suivre, donnée uniquement à titre d'exemple non limitatif et faite en référence aux dessins annexés sur lesquels :
- [Fig.1] la figure 1 est une vue schématique d'un système de caractérisation selon l'invention ;
- [Fig.2] la figure 2 est un organigramme d'un procédé de caractérisation selon l'invention, le procédé étant mis en œuvre par le système de caractérisation de la figure 1 ; et
- [Fig.3] [Fig.4] les figures 3 et 4 sont différentes vues illustrant la mise en œuvre du procédé de caractérisation de la figure 2.

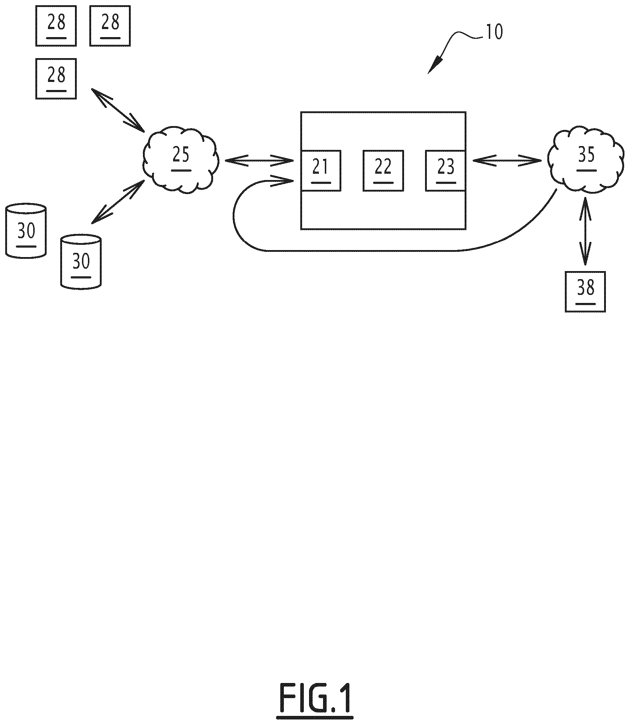

On a en effet illustré sur la figure 1 un système de caractérisation 10 de routes aéronautiques.

Dans tout ce qui suit, par route aéronautique, on entend un enchainement de vols effectués par un même opérateur.

Par vol, on entend par la suite un déplacement d'un aéronef à l'aide de l'opérateur à partir d'un aéroport de départ jusqu'à un aéroport d'arrivée.

Par mission, on entend un ou plusieurs vols effectués ou devant être effectués par l'opérateur.

Par aéronef, on entend tout engin volant qui est pilotable par l'opérateur à partir d'un cockpit de celui-ci (c'est le cas notamment d'un avion, par exemple d'un avion de ligne ou alors d'un hélicoptère) ou alors à distance (c'est notamment le cas d'un drone).

Par opérateur, on entend donc un pilote ou un co-pilote pilotant l'aéronef à partir du cockpit de celui-ci ou à distance ou encore un personnel navigant commercial officiant dans la cabine de l'aéronef.

Le système de caractérisation 10 permet de caractériser les routes aéronautiques effectuées par une population d'opérateurs.

La population d'opérateurs inclut plus que deux opérateurs, par exemple quelques dizaines d'opérateurs. Dans certains exemples, la population d'opérateurs inclut quelques centaines d'opérateurs, voire plus. La population d'opérateurs peut varier en fonction de critères de filtrage qui seront expliqués plus en détail par la suite.

En référence à la figure 1, le système de détermination 10 comprend un module d'entrée 21, un module de traitement 22 et un module de sortie 23.

Chacun de ces modules 21 à 23 se présente par exemple au moins partiellement sous la forme d'un logiciel et/ou alors d'un circuit logique programmable tel qu'un circuit FPGA (« Field Programmable Gate Array »).

Lorsque ces modules se présentent au moins partiellement sous la forme de logiciels, le système de détermination 10 comprend en outre un processeur permettant de mettre en œuvre ces logiciels et une mémoire vive permettant de stocker au moins temporairement les données à traiter ou alors les données traitées par ces différents modules. Le système de détermination 10 peut également comprendre une mémoire non volatile permettant de stocker au moins certaines données d'entrée ou alors des données de sortie, au moins de manière temporaire.

Le module d'entrée 21 est configuré pour recevoir des données issues de systèmes externes.

Dans l'exemple de la figure 1, les systèmes externes comprennent notamment une pluralité de systèmes transportables 28 d'évaluation de la fatigue ainsi qu'une ou plusieurs base(s) de données 30.

Chaque système transportable 28 d'évaluation de la fatigue permet de générer des pluralités de données d'évaluation relatives à la fatigue des différents opérateurs.

En particulier, chacun des systèmes transportables 28 d'évaluation permet de générer les données d'évaluation des opérateurs à partir des données physiologiques de ces opérateurs.

Les données physiologiques de l'opérateur présentent tout type de données permettant de caractériser l'état physique de l'opérateur. Ces données physiologiques sont avantageusement acquises juste avant la mission (i.e. vol) lors d'une phase initiale de collecte, ou pendant la mission (i.e. vol) lors d'une phase intermédiaire de collecte, ou alors juste après la mission (i.e. vol) lors d'une phase finale de collecte.

Avantageusement, les données physiologiques de l'opérateur comprennent au moins un type de données choisi dans le groupe comprenant :
- des images ou des vidéos de l'opérateur ;
- un rythme cardiaque ;
- une pression artérielle ;
- une inspiration d'oxygène ;
- une fréquence de respiration ;
- une amplitude de respiration ;
- une sudation ;
- une saturation en oxygène ;
- un taux de déshydratation.

Pour acquérir les données physiologiques, chaque système transportable 28 d'évaluation comprend une pluralité de capteurs. Alternativement ou avantageusement, chaque système transportable 28 d'évaluation est connecté directement ou indirectement à une pluralité de capteurs disposés par exemple dans le poste de travail de l'opérateur. Par exemple, ces capteurs sont disposés de manière fixe et/ou amovible dans le cockpit de l'aéronef piloté par l'opérateur.

En particulier, la pluralité de capteurs comprend tout capteur permettant d'acquérir les données physiologiques de l'opérateur.

Par exemple, la pluralité de capteurs comprend une caméra configurée pour acquérir des images de l'opérateur et un capteur de rythme cardiaque permettant de mesurer le rythme cardiaque de l'opérateur.

La caméra est par exemple orientée vers l'opérateur ou présente des moyens permettant de l'orienter selon la position de l'opérateur.

Le capteur de rythme cardiaque de l'opérateur est configuré par exemple pour être positionné autour d'un poignet de l'opérateur.

À cet effet, le capteur de rythme cardiaque présente par exemple une montre connectée ou un bracelet susceptible d'être fixé(e) sur le poignet de l'opérateur et une partie sensible qui est destinée à mesurer le rythme cardiaque de l'opérateur lorsque le bracelet est fixé sur son poignet.

La mesure du rythme cardiaque s'effectue par exemple par la partie sensible, par la technique appelée photopléthysmographie, dit PPG. Alternativement, la partie sensible est configurée pour effectuer la mesure du rythme cardiaque à partir d'une analyse de réponse électrique par le poignet de l'opérateur ou par analyse de signaux de radar se propageant dans le poignet de l'opérateur.

Dans certains exemples, le capteur de rythme cardiaque est configuré pour mesurer d'autres paramètres physiologiques de l'opérateur tels que (liste non exhaustive) la pression artérielle, l'inspiration de l'oxygène, la fréquence de respiration, l'amplitude de respiration la sudation, le taux de déshydratation.

Pour la saturation en oxygène, le capteur de rythme cardiaque est par exemple configuré pour émettre en direction de la peau de l'opérateur et recevoir un signal lumineux comprenant au moins deux longueurs d'onde. Une première longueur d'onde correspondant à une longueur d'onde absorbée par les globules rouges saturés, une deuxième longueur d'onde correspondant à une longueur d'onde absorbée par les globules rouges non saturés. Pour déterminer la saturation en oxygène, le capteur de rythme cardiaque est alors configuré pour comparer l'intensité lumineuse reçue en réponse à chacune des deux longueurs d'onde.

De manière générale, le capteur de rythme cardiaque peut se présenter sous la forme d'une montre connectée pour par exemple mesurer son rythme cardiaque.

Bien entendu, les fonctionnalités précitées du capteur de rythme cardiaque peuvent former des capteurs séparés.

Les données d'évaluation transmises par les systèmes transportables 28 d'évaluation comprennent avantageusement des niveaux de fatigue objectifs des opérateurs ayant utilisé ces systèmes 28.

De préférence, ces données d'évaluation comprennent en outre des niveaux de fatigue subjectifs de ces opérateurs.

En particulier, chaque niveau de fatigue objectif est déterminé au moins partiellement par le système transportable d'évaluation 28 à partir des données physiologiques de l'opérateur et éventuellement des données contextuelles. Dans certains exemples, les niveaux de fatigue objectifs sont déterminés par un ou plusieurs systèmes distants des systèmes transportables 28 d'évaluation à partir par exemple des données physiologiques des opérateurs transmises par ces systèmes 28. Ce ou ces systèmes distants peuvent former des serveurs.

Chaque niveau de fatigue subjectif de l'opérateur est saisi par l'opérateur lui-même via par exemple une interface du système transportable 28 d'évaluation correspondant.

De manière avantageuse, les systèmes transportables 28 sont aptes en outre à fournir des données de contexte général relatives au contexte général d'évaluation des opérateurs.

Ces données de contexte général comprennent au moins un type de données choisi dans le groupe comprenant :
- les données relatives à l'environnement de l'opérateur ;
- les données physiologiques de l'opérateur.

Les données relatives à l'environnement de l'opérateur sont par exemple des données décrivant l'environnement dans lequel l'évaluation de l'opérateur a été faite.

Les données physiologiques de l'opérateur sont relatives à l'opérateur lui-même et comprennent par exemple des données déterminées par les différents capteurs tels qu'expliqués précédemment.

Dans certains cas, ces données physiologiques comprennent également des données physiologiques saisies par l'opérateur via l'interface de communication du système transportable 28 d'évaluation correspondant. Ces données sont saisies par exemple par l'opérateur à la suite de différentes questions relatives à son état physiologique général tel que, par exemple, la durée de son sommeil, la quantité de sieste effectuée, les heures de ou des périodes de repos, etc.

Les données de contexte général sont par exemple liées aux données d'évaluation transmises par le système transportable 28 d'évaluation correspondant par un identifiant unique de session.

Autrement dit, cet identifiant unique de session permet d'associer les données d'évaluation déterminées par ce système 28 aux données de contexte général qui correspondent à ces données d'évaluation.

Cet identifiant unique de session peut par exemple être associé à un identifiant de l'opérateur dont les données d'évaluation sont utilisées. Pour ce faire, les données de contexte général peuvent inclure l'identifiant de cet opérateur. L'identifiant de l'opérateur peut éventuellement être anonymisé.

La ou les base(s) de données 30 permettent de fournir des données de contexte particulier. Ces données de contexte particulier comprennent au moins un type de données choisi dans le groupe comprenant :
- les données relatives à la mission à effectuer par l'opérateur ;
- les données relatives à la ou aux mission(s) effectuées par l'opérateur ;
- les données opérationnelles relatives à des activités effectuées par l'opérateur autres qu'une mission.

Ces données de contexte particulier correspondent par exemple au planning de vol effectué ou à effectuer par différents opérateurs.

Les données sur des activités autres qu'une mission effectuées par les opérateurs comprennent par exemple des données sur leurs activités physiques. Ces données sont par exemple issues d'une application de suivi sportif de l'opérateur ou de tout autre organisme s'occupant des activités des opérateurs.

Les activités effectuées par l'opérateur peuvent comprendre en outre par exemple des activités en vol ou au sol telles qu'entrainement, astreinte, maladie, etc.

La ou les base(s) de données 30 peuvent alors appartenir à la compagnie aérienne ou à toute autre organisme tiers qui stocke les données de contexte particulier telles que définies ci-dessus.

Le module de traitement 22 permet de traiter les données acquises par le module d'entrée 21 comme cela sera expliqué plus en détail par la suite.

Le module de traitement 22 permet en outre de générer des données de sortie qui sont transmises au module de sortie 23.

Le module de sortie 23 permet d'envoyer les données générées par le module de traitement 22 à tout système externe intéressé. Ce système externe est par exemple raccordé à ce module de sortie 23 via un réseau informatique global ou local 35.

Dans l'exemple de la figure 1, un tel système externe comprend par exemple une interface de communication 38 avec un utilisateur tel qu'un manager (i.e. par exemple *Safety Manager* en anglais) ou tout autre supérieur des opérateurs.

Cette interface de communication 38 comprend par exemple un moyen d'affichage tel qu'un écran et un moyen de saisie.

Ce moyen de saisie permet par exemple à l'utilisateur de saisir un critère d'affichage qui peut être également transmis au module d'entrée 21 pour être pris en compte dans les données de sortie transmises par le module de sortie 23.

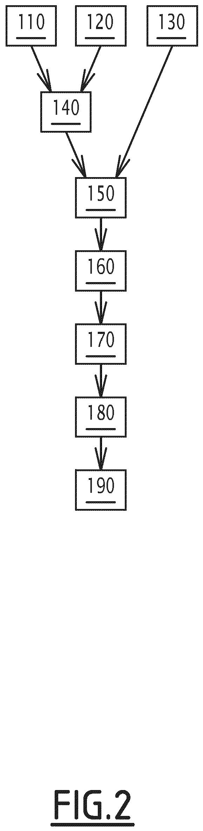

Le système de caractérisation 10 est configuré pour mettre en œuvre un procédé de caractérisation qui sera désormais expliqué plus en détail en référence à la figure 2 présentant un organigramme de ses étapes.

Il est considéré initialement que les systèmes transportables 28 d'évaluation ont généré des données physiologiques relatives à une population d'opérateurs. Ces données physiologiques sont utilisées par les systèmes transportables 28 et/ou d'autres systèmes distants pour générées des données d'évaluation.

Les données d'évaluation comprennent notamment les niveaux de fatigue objectifs/subjectifs de ces opérateurs.

De manière avantageuse, les données d'évaluation ont été générées suite à une ou plusieurs phases de collecte.

Comme indiqué précédemment, chaque phase de collecte est choisie parmi une phase initiale de collecte mise en œuvre avant la mission, une phase intermédiaire de collecte mise en œuvre pendant la mission et une phase finale de collecte mise en œuvre après la mission.

La phase initiale de collecte, appelée également check-in, comprend l'acquisition de données physiologiques et de données de mission relatives à l'opérateur correspondant et génère un niveau de fatigue objectif à partir de ces données.

La phase intermédiaire de collecte, appelée également on-duty, comprend une collecte de différents types de données telles que les données physiologiques de l'opérateur et des données relatives à la mission en cours. Cette collecte par exemple est effectuée par un dispositif distant du dispositif transportable d'évaluation. Un tel dispositif distant peut comprendre une montre connectée ou tout autre dispositif mobile porté par l'opérateur pendant la mission.

La phase finale de collecte, appelée également check-out, comprend la collecte de données générées pendant la mission ainsi que les données physiologiques de l'opérateur acquises par le système transportable d'évaluation 28 suite à la mission.

Dans certains modes de réalisation, la phase intermédiaire de collecte est optionnelle. Dans un tel cas, seules les phases initiale et finale de collecte sont mises en œuvres.

Il est également considéré qu'initialement les systèmes transportables 28 d'évaluation génèrent les données de contexte général associées aux données d'évaluation des opérateurs. Ces données de contexte général sont par exemple liées aux données d'évaluation de l'opérateur correspondant par un identifiant unique de session tel que défini précédemment.

Enfin, il est également considéré que la ou les base(s) de données 30 contiennent les données de contexte particulier relatives au contexte particulier d'évaluation des opérateurs.

Lors des étapes 110, 120, 130, le module d'entrée 21 acquiert respectivement les données d'évaluation, les données de contexte général et les données de contexte particulier.

Ces étapes 110 à 130 sont par exemple mises en œuvre de manière parallèle. En variante, au moins certaines de ces étapes sont mises en œuvre de manière consécutive.

Dans certains modes de réalisation, les données de contexte général ne sont pas nécessaires. Dans un tel cas, l'étape 120 d'acquisition de ces données n'est alors pas mise en œuvre.

À la fin de chacune des étapes 110 à 130, les données correspondantes sont transmises par le module d'entrée 21 au module de traitement 22.

Lors d'une étape suivante 140, le module de traitement 22 met en œuvre un prétraitement des données d'évaluation et des données de contexte général.

Le prétraitement est par exemple choisi en fonction de la nature des données acquises.

Ainsi, par exemple, le prétraitement des données de contexte général comprend la mise en œuvre d'au moins l'un des éléments choisis dans le groupe comprenant :
- la définition de la localisation de l'évaluation (par exemple à partir de la configuration ou à partir des coordonnées géographiques de l'évaluation) ;
- la définition de l'heure locale (calculée par exemple en fonction de la localisation de l'évaluation) ;
- l'identification d'une session matinale, normale ou tardive ;
- l'identification du poste occupé par l'opérateur pendant sa mission (par exemple pilote ou co-pilote) ;
- l'extrapolation des informations relatives au sommeil de l'opérateur (par exemple durée de sommeil, les heures de couchée-levée, durée de sieste, chronotype, etc.).

En particulier, en ce qui concerne l'identification de la session matinale, normale ou tardive, les vols matinaux ou tardifs sont définis par la règlementation (par exemple ORO.FTL.105, (i) and ARO.OPS.230). Pour les sessions, on ajoute une marge de deux heures avant le départ du vol (puisque le pilote arrive largement avant le vol) et une heure après l'arrivée du vol. De ce fait, la plage matinale se situe par exemple entre 03h00 et 05h59, et la plage tardive se situe entre 23h00 et 02h59 en heure locale de la localisation de l'opérateur.

Le prétraitement des données d'évaluation comprend par exemple la mise en œuvre d'au moins l'un des éléments choisis dans le groupe comprenant :
- la normalisation du niveau de fatigue (par exemple sur l'échelle de KSS (« Karolinska Sleepiness Scale ») variant de 1 à 9) ;
- la normalisation du niveau de fatigue subjectif (par exemple selon la même échelle de KSS) ;
- l'identification d'une classe de fatigue objective selon le niveau de fatigue objectif ;
- l'identification d'une classe de fatigue subjective selon le niveau de fatigue subjectif (selon par exemple les mêmes classes que celles relatives au niveau de fatigue objectif).

La classification du niveau de fatigue en différentes classes peut s'effectuer par exemple en fonction de la valeur du niveau de fatigue objectif ou subjectif. Le nombre de classes présente par exemple une valeur prédéterminée qui peut par exemple être choisie entre 2 et 10. Ainsi, par exemple, il est possible de choisir uniquement deux classes de fatigue (satisfaisante et insatisfaisante) ou trois classes de fatigue (intermédiaire, élevée, très élevée).

À cet effet, le module de traitement 22 peut par exemple comparer le niveau de fatigue objectif subjectif déterminé et éventuellement normalisé avec des seuils prédéterminés.

Lors de l'étape 150 suivante, le module de traitement 22 met en œuvre une extraction à partir des données de contexte particulier, des données sur des vols effectués par les opérateurs.

En particulier, le module de traitement 22 peut associer à chaque identifiant d'opérateur pour lequel les données d'évaluation sont disponibles des données sur des vols effectués par l'opérateur correspondant à cet identifiant.

Puis, à partir de ces données de vols effectués, le module de traitement 22 identifie une pluralité de routes aéronautiques effectuées par les opérateurs correspondants.

Selon un exemple particulier de l'invention, pour cela, le module de traitement 22 exploite les données stockées dans la table de vols opérés issue de la ou des bases de données 30.

Si ces données sur les vols opérés contiennent pour une session un aéroport de départ et un aéroport d'arrivée, le module de traitement 22 construit la route aéronautique correspondante en parcourant la liste ordonnée des différents vols de cette session et en concaténant les aéroports de départ et d'arrivée.

En particulier, la liste ordonnée des différents vols peut se présenter sous la forme suivante :

| | | |
|---|---|---|
| Leg 0 : | departure_airport = DEP₁ | arrival_airport = ARR₁ |
| Leg 1 : | departure_airport = ARR₁ | arrival_airport = ARR₂ |
| ... .. | | |
| Leg n-1 : | departure_airport = ARRₙ₋₂ | arrival_airport = ARRₙ₋₁ |
| Leg n : | departure_airport = ARRₙ₋₁ | arrival_airport = ARRₙ |

où
- Leg i désigne un vol associé à l'indice i ;
- DEPᵢ désigne l'aéroport de départ associé à l'indice i ; et
- ARRᵢ désigne l'aéroport d'arrivée associé à l'indice i.

Le module de traitement 22 s'assure que l'aéroport de départ du vol ayant l'indice i est égal à l'aéroport d'arrivée du vol ayant l'indice i-1.

En absence de discontinuité, la route suivante peut alors être construite : DEP₁-ARR₁- ARR₂- ...... - ARRₙ₋₂- ARRₙ₋₁- ARRₙ.

Lorsqu'une discontinuité est détectée sur la route, le module de traitement 22 rejette alors les données correspondantes de la future considération.

Si pour les vols opérés pour une session ne contiennent pas d'aéroport de départ ni d'arrivée, le module de traitement 22 construit la route en parcourant la planification de vols (appelée « fiche de Rostering ») de l'opérateur considéré. Puis, à partir de cette planification, le module de traitement 22 reconstitue les aéroports de départ et les aéroports d'arrivée et ensuite fait la concaténation des aéroports de départ et d'arrivée de la même façon qu'expliquée précédemment.

Lors de l'étape suivante 160, le module de traitement 22 fait la corrélation entre les données acquises lors de différente phase de collecte.

Par exemple, dans certains cas, il existe des données qui sont acquises ou déterminées uniquement lors de la phase initiale de collecte et certaines autres données qui sont acquises/déterminées uniquement lors de la phase finale de collecte.

Dans un tel cas, le module de traitement 22 va associer ces données en utilisant l'identifiant unique de session associé à chaque type de données et l'identifiant de l'opérateur. Par exemple, lorsque le module de traitement 22 identifie un même identifiant de l'opérateur pour deux identifiants uniques de session correspondant respectivement à une phase initiale de collecte et à une phase finale de collecte, il peut faire une corrélation entre les données collectées lors de ces différentes phases.

Il est de même en ce qui concerne la phase intermédiaire de collecte.

De plus, il est possible de corréler les données issues de plusieurs phases initiales de collecte et de plusieurs phases finales de collecte relatives au même opérateur.

Lors de l'étape suivante 170, le module de traitement 22 détermine un indicateur de fatigue pour chaque route aéronautique identifiée.

Cet indicateur de fatigue est par exemple déterminé en fonction des données d'évaluation des opérateurs ayant effectué cette route.

Pour cela, le module de traitement 22 détermine d'abord un niveau de fatigue subjective ou objective d'un opérateur ayant effectué cette route puis fait une sommation pondérée des niveaux de fatigue des opérateurs différents ayant effectué cette route.

La pondération s'effectue en utilisant des coefficients de pondération qui sont déterminés par exemple en fonction des règles prédéterminées. Ces règles prédéterminées sont par exemple déterminées par la compagnie aérienne en charge des opérateurs.

Selon certains modes de réalisation, l'indicateur de fatigue associé à une route donnée est déterminé en outre en prenant en compte des données de contexte général qui forme un critère de filtrage. Ainsi, plusieurs indicateurs de fatigue peuvent être déterminés pour différentes données de contexte général.

Par exemple, un indicateur de fatigue pour une route aéronautique donnée peut être déterminé pour une tranche d'âge d'opérateurs prédéterminée ou par exemple pour des vols matinaux ou alors pour des vols tardifs.

Autrement dit, différents critères de filtrage peuvent être utilisés pour déterminer l'indicateur de fatigue sur une route particulière. Ces critères de filtrage sont notamment basés sur différentes données de contexte général.

Dans certains modes de réalisation, le procédé peut comprendre en outre une étape 180, lors de laquelle, le module de traitement 22 détermine un indicateur de fatigue pour chaque vol d'une même route aéronautique en fonction du niveau de fatigue objectif/subjectif déterminé pour différents opérateurs pour ce vol.

Pour cela, le module de traitement 22 peut sélectionner une route et extraire les niveaux de fatigue mesurés en vol pour les associer à chacun des vols opérés. Les niveaux de fatigue relatifs aux différents opérateurs peuvent ensuite être analysés statistiquement pour déterminer un niveau de fatigue objectif/subjectif pour chaque vol.

Lors de l'étape suivante 190, le module de traitement 22 transfère les données déterminées au module de sortie 23 qui les transmet alors à l'interface 38 pour par exemple être représentées à l'utilisateur.

Ces données sont par exemple représentées sous la forme de diagrammes associés à différentes routes aéronautiques.

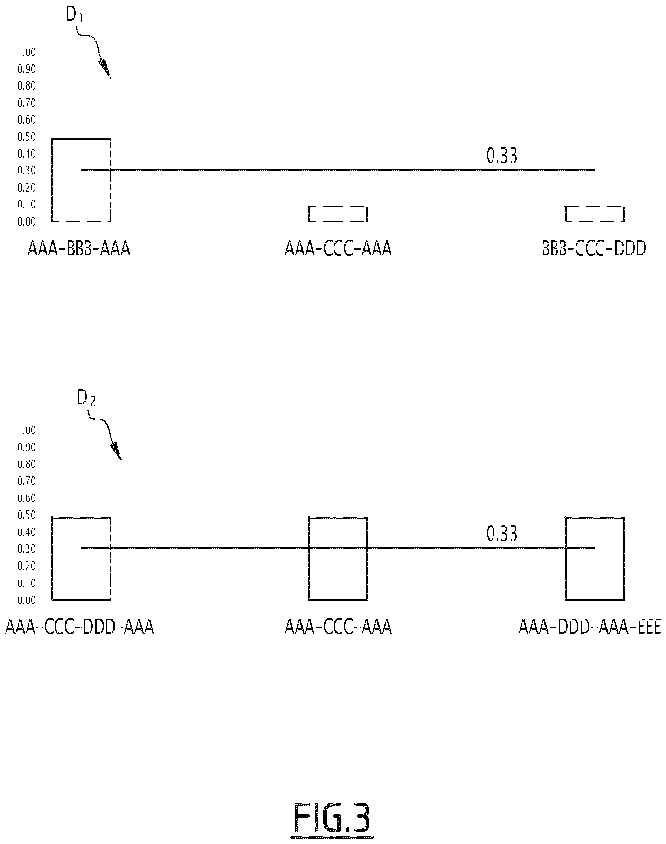

La figure 3 illustre un exemple de visualisation de tels diagrammes.

En particulier, la figure 3 illustre deux diagrammes, à savoir le diagramme D1 et le diagramme D2, présentant respectivement les indicateurs de fatigue déterminés en fonction des niveaux de fatigue objectifs et subjectifs en relation avec trois routes.

Ces trois routes sont marquées sur cette figure 3 par l'annotation AAA-BBB-AAA, AAA-CCC-AAA et BBB-CCC-DDD.

De plus, dans chacun des cas, l'indicateur de fatigue déterminé peut être comparé avec par exemple un seuil déterminé par la compagnie aérienne.

Ainsi, comme cela est montré sur le diagramme D1, l'indicateur de fatigue seulement de la première route dépasse le seuil prédéterminé alors que les indicateurs des deux autres routes se trouvent au-dessous de ce seuil.

En revanche, selon le diagramme D2, les indicateurs de fatigue des trois routes dépassent le seuil prédéterminé.

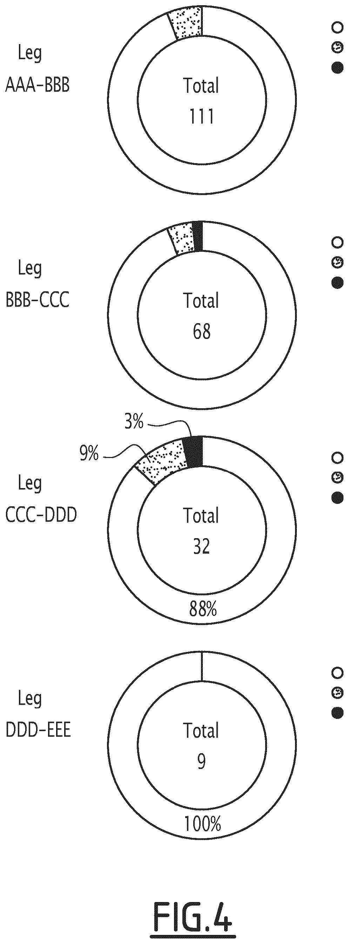

La figure 4 illustre un exemple de visualisation des indicateurs de fatigue de différents vols d'une même route.

Cette même route est formée alors de quatre vols à savoir les vols AAA-BBB, BBB-CCC, CCC-DDD et DDD-EEE.

Les niveaux de fatigue sur chacun de ces vols peuvent être classés en trois classes, telles que déterminées précédemment. Selon cette figure 4, il est donc clair que le vol le plus fatiguant est le vol CCC-DDD.

Bien entendu, de nombreuses autres visualisations de diagramme en fonction des différentes données de contexte général sont également possibles.

On conçoit alors que la présente invention présente un certain nombre d'avantages. Tout d'abord, l'invention permet de simplifier la planification de vol par exemple par le manager de sécurité *(Safety Manager)* en utilisant les indicateurs de fatigue relatifs à chaque route.

Ainsi, les managers peuvent comparer ces différents indicateurs pour déterminer l'enchainement de vols moins fatiguant pour les opérateurs.

La sécurité des vols peut ainsi être améliorée.

## Revendications

1. Procédé de caractérisation de routes aéronautiques, chaque route aéronautique présentant un enchainement de vols effectués par un même opérateur, chaque vol étant défini par un aéroport de départ et un aéroport d'arrivée, le procédé comprenant les étapes suivantes :
- acquisition (110) d'une pluralité de données d'évaluation relatives à une population d'opérateurs, les données d'évaluation étant déterminées à partir de données physiologiques des opérateurs ;
- prétraitement (140) des données d'évaluation ;
- acquisition (130) d'une pluralité de données de contexte particulier relatives au contexte particulier d'évaluation des opérateurs ;
- extraction (150) à partir des données de contexte particulier, de données sur des vols effectués par les opérateurs et identification d'une pluralité de routes aéronautiques correspondantes ;
- pour chaque route aéronautique identifiée, détermination (170) d'un indicateur de fatigue en fonction des données d'évaluation des opérateurs ayant effectué cette route.

2. Procédé selon la revendication 1, dans lequel les données d'évaluation comprennent au moins un type de données choisi dans le groupe comprenant :
- niveau de fatigue objectif de l'opérateur ;
- niveau de fatigue subjectif de l'opérateur.

3. Procédé selon la revendication 2, lequel le prétraitement des données d'évaluation comprend la mise en œuvre d'au moins l'un des éléments choisi dans le groupe comprenant :
- normalisation du niveau de fatigue objectif ;
- normalisation du niveau de fatigue subjectif ;
- identification d'une classe de fatigue objective selon le niveau de fatigue objectif ;
- identification d'une classe de fatigue subjective selon le niveau de fatigue subjectif

4. Procédé selon la revendication 2 ou 3, dans lequel la détermination de l'indicateur de fatigue pour chaque route aéronautique comprend la détermination d'une somme pondérée des niveaux de fatigue des opérateurs ayant effectué cette route.

5. Procédé selon la revendication 4, dans lequel les coefficients de pondération sont déterminés selon des règles prédéterminées.

6. Procédé selon l'une quelconque des revendications précédentes, comprenant en outre une étape de corrélation (160) de données acquises/déterminées lors de différentes phases de collecte réalisées en relation avec un même opérateur, chaque phase de collecte étant choisie parmi une phase initiale de collecte mise en œuvre avant la mission, une phase intermédiaire de collecte mise en œuvre pendant la mission et une phase finale de collecte mise en œuvre après la mission.

7. Procédé selon l'une quelconque des revendications précédentes, comprenant en outre une étape acquisition (120) d'une pluralité de données de contexte général relatives au contexte général d'évaluation des opérateurs, ledit étape de prétraitement (140) comprenant en outre un prétraitement de ces données de contexte général.

8. Procédé selon l'une quelconque des revendications précédentes, dans lequel les données de contexte général comprennent au moins un type de données choisi dans le groupe comprenant :
- données relatives à l'environnement de l'opérateur ;
- données physiologiques de l'opérateur.

9. Procédé selon la revendication 8, dans lequel le prétraitement des données de contexte général comprend la mise en œuvre d'au moins l'un des éléments choisi dans le groupe comprenant :
- définition de la localisation de l'évaluation ;
- définition de l'heure locale ;
- identification d'une session matinale, normale ou tardive ;
- identification du poste occupé par l'opérateur ;
- extrapolation des informations relatives au sommeil de l'opérateur.

10. Procédé selon l'une quelconque des revendications 7 à 9, dans lequel l'étape de détermination (170) d'un indicateur de fatigue pour chaque route aéronautique comprend en outre la détermination de différents indicateurs de fatigue associées à cette route aéronautique en fonction de différentes données de contexte général formant un ou plusieurs critères de filtrage.

11. Procédé selon l'une quelconque des revendications précédentes, comprenant en outre une étape (180) de détermination d'un indicateur de fatigue pour chaque vol d'une même route aéronautique en fonction de niveaux de fatigue objectifs/subjectifs déterminés pour différents opérateurs pour ce vol.

12. Système de caractérisation (10) de routes aéronautiques, comprenant des moyens techniques (21, 22, 23) configurés pour mettre en œuvre le procédé selon l'une quelconque des revendications précédentes.
